# EUROPEAN PATENT SPECIFICATION

(11) **EP 0 835 089 B1**
(45) Date of publication and mention of the grant of the patent: **25.09.2002**
(21) Application number: 96922601.8
(22) Date of filing: 27.06.1996
(51) Int. Cl.: A61F 13/15, A61F 13/20

(54) **ODOR CONTROL DEVICE**
VORRICHTUNG ZUR GERUCHSKONTROLLE
DISPOSITIF D'ELIMINATION DE MAUVAISES ODEURS

(30) Priority: 27.06.1995 IT TO950541
(43) Date of publication of application: 15.04.1998
(73) Proprietor: THE PROCTER & GAMBLE COMPANY, Cincinnati, Ohio 45202 (US)
(72) Inventor: CARLUCCI, Giovanni, I-66100 Chieti (IT); CINTIO, Achille, Di, I-65126 Pescara (IT)
(74) Representative: Hirsch, Uwe Thomas
(86) International application number: US9611003
(87) International publication number: WO97001317

(56) References cited:
- EP-A- 0 523 719
- US-A- 5 407 442

## Description

This invention relates to an improved article for absorbing bodily fluid and controlling the emission of odors from the absorbed fluids, and in particular to an improved article which comprises an odor control material in combination with a perforated film topsheet.

Absorbent articles are designed to be worn by humans to absorb bodily fluids, such as urine, menstrual fluid, perspiration, etc. Examples of absorbent articles include sanitary napkins, pantiliners, disposable diapers, incontinence pads, tampons and the like.

In use, the absorbent articles are known to acquire a variety of compounds, for example volatile fatty acids (e.g. isovaleric acid), ammonia, amines (e.g. triethylamine), sulphur-containing compounds (e.g. mercaptans, sulphides), alcohols, ketones and aldehydes (e.g. furaldehyde) which can release unpleasant odors. These compounds may be present in the bodily fluid or may be produced by fermentation once the bodily fluid is absorbed into the article. In addition bodily fluids can contain microorganisms that can also generate malodorous by products. Unpleasant odors which emanate from absorbent pads when in use may make the wearer feel self conscious.

As noted above, a variety of absorbent products are well known items of commerce and everyday life. Sanitary napkins and pantiliners are typical examples. A pantiliner, for example, normally comprises a topsheet, an absorbent element, and a backsheet. The topsheet is that part of the device which, in use, contacts the body of the user. Fluids discharged from the body normally contact the topsheet first, pass through it and are retained in the article by the absorbent element. The topsheet, then is normally a porous planar material while the absorbent element is any convenient structure which will absorb fluids. The backsheet, which is optional but highly preferred, prevents absorbed fluids from escaping from the article and soiling the users garments. Odor controlling absorbent articles normally comprise an odor control system, such as a chemical compound or mixture of compounds. This odor control system is frequently distributed throughout the absorbent element.

Various odor controlling products and odor controlling materials have been disclosed. For example, WO 81/01643 discloses the removal of ammonia from diapers by incorporating into the diaper an inorganic aluminosilicate zeolite ammonium ion exchange material.

Also, US 4 826 497 discloses fibrous absorbent articles intended for the absorption of bodily fluids comprising synthetic zeolites prepared using an organic templating agent prepared in siliceous form in which at least about 90% of the framework tetrahedral units are SiO₂ tetrahedra, which has pore diameters of at least 5.5. A and has a capacity for absorbed water of not greater than 10% under standard conditions. In this specification, the decimal is represented by the point.

US 5 407 442 discloses odour controlling compositions comprising carbon particles combined with white odor-controlling agents by means of binders as well as absorbent articles comprising such compositions.

Italian Patent Application TO94A000227 (WO 95/26207) describes an exemplary odor control system based on zeolite A, and more particularly on sodium zeolite A having an SiO₂/Al₂O₃ ratio of less than 4:1. This odor control system will be discussed more fully below.

The prior art also discloses the use of various planar materials as topsheet. For example, nonwoven fabrics of natural synthetic fibers have been used extensively. Apertured polymeric films have also been used in absorbent sanitary articles. In particular, EP 0 523 719, EP 0 018 020 issued to Radel and Thompson and EP 0 059 509 issued to Ahr et al disclose an exemplary formed polymeric film topsheets.

Despite the advances made in the field of absorbent articles in recent years, the search for articles having improved performance, such as improved odor control performance, has continued.

Accordingly, it is an object of the present invention to provide an absorbent article which will control the emission of odors to a greater degree than will previous articles.

It has been surprisingly discovered that the use of an apertured polymeric film topsheet as claimed in claim 1 in combination with an effective chemical-based odor control system will result in a more effective odor controlling absorbent article.

### Odor Control System

The present invention provides an absorbent article having incorporated therein an odor control system (or material) for decreasing odors associated with bodily fluids. Any effective and safe odor control system can be used in the present invention. Those mentioned above are examples.

It has been found, however, that the system mentioned in Italian Application TO94A000227 is especially useful. Preferably the size of the zeolite is from 200 to 500µm, more preferably from 300µm to 400µm.

The zeolite may be used as such or in a form which has been granulated with a binder.

While naturally occurring zeolites can be used, synthetic zeolites of the types available in commerce are generally preferred.

In general terms, zeolites comprise an aluminate/silicate framework, with associated cations, M, providing overall electrical neutrality.

Empirically, the zeolite framework can be represented as

X AlO₂ . y SiO₂

and the electrical neutral zeolite as

x/n M. x AlO₂ . y SiO₂ . z H₂O

wherein: x and y are each integers, M is a cation and n is the charge on the cation. As noted by the empirical formula, zeolites may also comprise water of hydration (z H₂O). reference to the literature will illustrate that M can be a wide variety of cation, e.g. Na⁺, K⁺, NH₄⁺, alkylammonium, heavy metals and the like. The sodium ion is convenient and preferred.

The manufacture of zeolite materials of the type used in the practice of this invention is well-known, and reference can be made of the literature for typical synthetic procedures. For example, attention is directed to synthetic procedures described in the following reference texts: ZEOLITE SYNTHESIS, ACS Symposium Series 398, Eds. M. L. Occelli and H. E. Robson (1989) pages 2 - 7; ZEOLITE MOLECULAR SIEVES, Structure, Chemistry and Use, by D. W. Breck, John Wiley & Sons (1974) pages 245 - 250, 313 - 314 and 348 - 352; MODERN APPLICATIONS OF MOLECULAR SIEVE ZEOLITES, Ph.D. Dissertation of S. M. Kuznicki, U. of Utah (1980), available from University Microfilms International, Ann Arbor, Michigan, pages 2 - 8.

The zeolite used in the present invention preferably has less than 10 %, preferably less than 7 % weight of particles having a size less than 100 µm.

The zeolite preferably has a low framework ratio, in particular it has a framework ratio of SiO₂/Al₂O₃ of not more than 4:1, preferably not more than 2:1.

The zeolite preferably has a pore diameter of from 0.30 to 0.55 nm, preferably from 0.4 to 0.45 nm.

The zeolite is preferably a zeolite A and more preferably a Na⁺ zeolite A.

The zeolite preferably is a compound based on a zeolite A which is granulated with sodium sulphate and carboxy methyl cellulose (CMC). This zeolite is sold by Degussa AG under the trade name Wessalith CS. The zeolite may also be granulated with bentonite and/or polyacrylate and is sold under the trade name Wessalith by Degussa AG. Although any zeolite having a particle size of at least 200 µm may be used in the practice of the invention.

The odor control system may also compose other conventional compounds, for example activated carbon, other zeolites; charcoal, antimicrobial agents, ionic absorbents, such as an absorbent gelling material (AGM) or known odor control agent.

It is desirable that the conventional compounds are also in powder form. These powders conventionally have particle sizes of at least 200 µm, preferably from 200 to 500 µm, thus enabling the odor control material to be easily handled and dosed with the conventional compound.

Preferably the odor control material also comprises AGM or charcoal, more preferably it comprises AGM and charcoal. The material may also comprise AGM and bentonite. The AGM, charcoal and bentonite all have odor controlling properties. The quantity of AGM, charcoal and bentonite used with the odor control material may readily be determined by the skilled person dependent on the absorbent article in question.

It has been found that from 2 to 70 % of the total weight of the absorbent article should be made up of the odor control material, which material may be zeolite alone or zeolite together with AGM and/or charcoal. For products having a weight of approximately 2 g a preferred quantity of odor control material is 0.7 g which quantity provides odor control. For 0.7 g of odor control material it is preferable to have about 31 % by weight of zeolite, about 43 % by weight AGM and about 26 % by weight charcoal.

As mentioned above, absorbent articles may be a sanitary napkin, a pantiliner, a disposable diaper, an incontinence pad, tampon or the like. According to one preferred aspect of the invention the absorbent article is a pantiliner. According to another preferred aspect of the invention the absorbent article is a sanitary napkin.

The weight of the odor control material which may be used in the absorbent article can be readily determined by the skilled person bearing in mind the size of the absorbent article in question. For example a suitable quantity of odor control material which may be used in a pantiliner is from 0.05 to 0.8 g, preferably the quantity is from 0.1 to 0.5 g.

Preferably AGM is included in the article together with the odor control material. The quantity of AGM which may be added may be readily determined by the skilled person for each absorbent article. Preferably 0.05 to 0.7 g and more preferably from 0.1 to 0.5 g of AGM is added to a pantiliner.

More preferably AGM and charcoal are included in the article together with the odor control material used in the present invention. The AGM is included in the amounts shown above and the charcoal is included in amounts of from 0.05 to 0.7 g, more preferably from 0.1 to 0.5 g in the pantiliner.

The odor control material may be incorporated into the article by methods known in the art, for example layered on the core of the absorbent material or mixed within the fibres of the absorbent core. The odor control material is preferably incorporated between two layers of cellulose tissue, optionally the material may be bonded between two cellulose tissue layers with, for example, a hot melt adhesive or any suitable bonding system.

More preferably the odor control material is incorporated in a layered structure in accordance with the disclosure of WO 94/01069 or
TO 93A 001028 (WO 95/17868) . TO 93A 001028 describes a layered structure substantially as described in WO 94/01069 with the exception that
TO 93A 001028 (WO 95/17868) comprises a much higher quantity of AGM in the intermediate layer which is between the fibrous layers, namely in an amount exceeding 120 g/m². In addition the intermediate layer in
TO 93A 001028 (WO 95/17868) also comprises thermoplastic material, which when heated, melts and bonds the first and second layers together with the intermediate layers and thus AGM between them. The bridges which form the bond points between the fibrous layers involve particles of AGM as well as particles of thermoplastic material. The absorbent capacity of the AGM is unaffected by bonding.

The second necessary component of the present invention is the apertured polymeric film topsheet.

The prior art notably the aforementioned Italian Application TO94A000277 (WO 95/26207) describes the attributes of topsheets suitable for use with , e. g. , the odor control system described therein. It has now been surprisingly discovered that apertured polymeric film topsheets according to claim 1, yield significantly better odor control than other types of topsheets such as, for example, thermal bonded nonwoven materials.

The term apertured polymeric topsheet as used herein refers to topsheets comprising at least one layer or a multiplicity of layers wherein at least one layer is formed from a continuous or uninterrupted film material wherein apertures are created.

In general the apertured polymeric topsheet of the present invention is compliant, soft feeling, and non-irritating to the wearer's skin. Further, the topsheet is fluid pervious permitting fluids (e.g., menses and/or urine) to readily penetrate through its thickness. Suitable apertured polmyeric film topsheets for use herein include polymeric apertured formed films, thermoplastic films, apertured plastic films, and hydroformed thermoplastic films; porous foams; net- like reticulated foams and thermoplastic scrims.

It has been further surprisingly discovered that three dimensional formed film topsheets as claimed in claim 1 provided better odor control than do conventional apertured film topsheets.

The formed film topsheet according to the invention has openings of two shapes: regular pentagons having an area of about 0.21 mm² and an irregular hexagon having an area of 1.78 mm². The openings are distributed so that the distance between the sides of the figures is about 0.37 mm to about 0.42 mm. The preforming and postforming film thickness may be, respectively 25 µm and 0.43 mm. This film may have an open area of about 33.7 %. Both films are made according to the teachings of the above-mentioned patent to Radel and Thompson.

The body surface of the formed film topsheet of the present invention may also be hydrophilic so as to help liquid to transfer through the topsheet faster than if the body surface was not hydrophilic. In this manner the likelihood that menstrual fluid will flow off the topsheet rather than flowing into and being absorbed by the absorbent structure is diminished. In a preferred embodiment, surfactant is incorporated into the polymeric materials of the formed film topsheet such as is described in U.S. Patent Application Serial No. 07/794,745, "Absorbent Article Having A Nonwoven and Apertured Film Coversheet" filed on November 19, 1991 by Aziz, et al. Alternatively, the body surface of the topsheet can be made hydrophilic by treating it with a surfactant such as is described in the above referenced U.S. 4,950,254.

As mentioned above, a pantiliner is an exemplary embodiment of an article into which the odor control material and topsheet of this invention may be incorporated.

The pantiliner may be of any shape known in the art, for example, rectangular, hour-glass, winged, etc.

An odor controlled pantiliner normally comprises a liquid pervious topsheet as described, an absorbent core, an odor control system and a liquid impervious backsheet. It is not, however, intended that the pantiliner should be limited to embodiments comprising only such elements. Additional elements known to the skilled person may also be included in the pantiliner. For example, a nonwoven material can be interposed between the perforated film topsheet and the absorbent core.

The inner surface of the topsheet, or of any secondary topsheet as known in the art, can be secured in contacting relation to the absorbent core. The topsheet can be maintained in contact with the absorbent core by applying adhesive, preferably in spaced limited areas. Examples of suitable adhesives used for such purpose include the acrylic emulsion E-1833BT manufactured by the Rohm & Haas company, Philadelphia, Pennsylvania and the acrylic emulsion WB 3805 manufactured by H.B. Fuller Company of St. Paul, Minnesota. The adhesives can be applied by any of the common techniques well-known to those skilled in the art or by the use of transfer rolls. The adhesive may be in the form of a uniform continuous layer, a patterned layer of adhesive, or an array of separate lines, spirals or spots of adhesive. The absorbent core is preferably secured in contacting relation to any secondary topsheet.

The absorbent core is positioned between the topsheet and the backsheet. The absorbent core provides the absorptive means for absorbing the bodily fluid. It is generally compressible, conformable and non-irritating to the user's skin. It can comprise any material used in the art for such purpose. Examples of such materials include multiple plies of creped cellulose wadding, fluffed cellulose fibres, wood pulp fibres also known as airfelt, textile fibres, a blend of fibres, a mass or batt of fibres, a web of polymeric fibres, a blend of polyester and polypropylene fibres, layers of cellulose tissue or layers of air laid tissue.

The core comprises a mass or batt of fibres while many types of fibres may be used, a preferred material is a batt of polyester fibres. More preferably the core comprises cellulose tissue (63 g/m²) which forms three absorbent layers.

Preferably, the odor control material disclosed herein is incorporated into the absorbent core by known techniques. It can, for example, be layered on the absorbent core or mixed with the fibres of the core. More preferably the odor control material is layered in accordance with the teaching of WO 94/01069 (corresponding to PCT/US93/06128) or TO93A001028 as discussed previously between two layers of air laid cellulose tissue.- In particular polyethylene powder, as thermoplastic material, is mixed with the odor control material of the present invention, preferably zeolite, AGM and charcoal, the mixture is heated such that the polyethylene melts and glues the laminate layers and components together. Polyethylene powder is preferably also placed on the edges of laminate to ensure that edges of the laminate stick together and any loose odor control material does not fall out of the laminate. Lines of hot melt adhesive are preferably placed on the edges of laminate to ensure that edges of the laminate stick together.

One especially, preferred backsheet material is a polyethylene film having a thickness of from about 1.9 µm to about 31.75 µm, with a 25.4 µm thickness polyethylene film being especially suitable. Preferably the backsheet is polyethylene embossed film (24.4 g/m²).

A layer of panty fastening adhesive is usually secured to the side of the backsheet opposite the absorbent core and is covered by a removable release liner. This panty fastening adhesive provides a means for securing the pantiliner in the crotch portion of a panty. Any adhesive or glue used in the art for such purpose can be used herein, with pressure sensitive adhesives being preferred. Suitable adhesives are Century A-305-IV manufactured by the Century Adhesives Corporation and Instant Lok 34-2823 manufactured by the National Starch Company. Also, before the pantiliner is placed in use, the panty fastening adhesive should be covered with a removable release liner in order to keep the adhesive from drying out or sticking to a surface other than the crotch portion of the panty prior to use. Any commercially available release liners commonly used for such purposed can be utilized herein. Non-limiting examples of suitable release liners are BL 30 MG-A Silox E1/0 and BL 30 MG-A Silox 4 P/0 both of which are manufactured by the Akrosil Corporation. Preferably the release liner is a silicon paper having a thickness of about 45 µm (43.5g/m²). Other means which are known in the art may be used to affix the pantiliner in the crotch portion of a panty.

The backsheet is preferably secured in the absorbent core by securement means well known in the art. Suitable securement means are the same means hereinbefore disclosed with respect to securing the optional secondary top sheet to the absorbent core.

## Claims

1. An absorbent article having incorporated therein an odor control material for decreasing the odor associated with bodily fluids, said article comprising a topsheet being a 3-dimensional formed polymeric film material, **characterized in that** said topsheet is provided with apertures in the form of regular pentagons having an open area in the plane of said topsheet of about 0.21 square millimeter and apertures in the form of irregular hexagons having an open area in the plane of the topsheet of about 1.78 square millimeters, the distances between any two of said apertures being from about 0.37 mm to about 0.42 mm.

2. An absorbent article as claimed in Claim 1 wherein said odor control material comprises a zeolite having an average particle size of at least 200 µm.

3. An absorbent article as claimed in Claim 2 wherein said zeolite is zeolite A.

4. An absorbent article as claimed in any of the preceding Claims wherein said odor control material additionally comprises AGM and charcoal.

## Patentansprüche

1. Absorbierender Artikel mit einem darin eingebauten Geruchsregulierungsmaterial zum Vermindern des mit Körperfluiden verbundenen Geruchs, wobei der Artikel aufweist eine Oberschicht, die ein dreidimensional geformtes, polymeres Filmmaterial ist, **dadurch gekennzeichnet, daß** die Oberschicht mit Öffnungen in Form regelmäßiger Fünfecke ist, die eine Öffnungsfläche in der Ebene der Oberschicht von etwa 0,21 Quadratmillimeter haben und mit Öffnungen in Form unregelmäßiger Sechsecke mit einer Öffnungsfläche in der Ebene der Oberschicht von etwa 1,78 Quadratmillimeter versehen ist, wobei die Abstände zwischen jeweils zwei der Öffnungen von etwa 0,37 mm bis etwa 0,42 mm beträgt.

2. Absorbierender Artikel nach Anspruch 1, in welchem das Geruchsregulierungsmaterial ein Zeolit mit einer mittleren Teilchengröße von wenigstens 200 µm umfaßt.

3. Absorbierender Artikel nach Anspruch 2, in welchem der Zeolit ein Zeolit A ist.

4. Absorbierender Artikel nach einem der vorstehenden Ansprüche, in welchem das Geruchtsregulierungsmaterial zusätzlich AGM und Holzkohle umfaßt.

## Revendications

1. Article absorbant renfermant un matériau de limitation des odeurs pour réduire les odeurs associées aux fluides corporels, ledit article comprenant une feuille de dessus qui est un matériau de film polymère formé, tridimensionnel, **caractérisé en ce que** ladite feuille de dessus est munie d'ouvertures sous la forme de pentagones réguliers ayant une étendue ouverte dans le plan de ladite feuille de dessus d'environ 0,21 mm² et des ouvertures sous la forme d'hexagones irréguliers ayant une étendue ouverte dans le plan de la feuille de dessus d'environ 1,78 mm², les distances entre l'une quelconque de deux desdites ouvertures étant comprises dans la gamme d'environ 0,37 mm à environ 0,42 mm.

2. Article absorbant selon la revendication 1, dans lequel le matériau de limitation des odeurs comprend une zéolite ayant une dimension de particule moyenne d'au moins 200 µm.

3. Article absorbant selon la revendication 2, dans lequel ladite zéolite est la zéolite A.

4. Article absorbant selon l'une quelconque des revendications précédentes, dans lequel ledit matériau de limitation des odeurs comprend, de façon additionnelle, un matériau gélifiant absorbant (AGM) et du charbon de bois.
